# FASCICULE DE BREVET EUROPEEN

(11) **EP 3 814 316 B1**
(45) Date de publication et mention de la délivrance du brevet: **04.05.2022**
(21) Numéro de dépôt: 19745716.1
(22) Date de dépôt: 26.06.2019
(51) Int. Cl.: C07C 67/08, C07C 67/54, C07C 67/58

(54) **PROCEDE DE PURIFICATION D'ACRYLATES LEGERS**
VERFAHREN ZUR REINIGUNG VON LEICHTEN ACRYLATEN
METHOD FOR PURIFYING LIGHT ACRYLATES

(30) Priorité: 27.06.2018 FR 1855753
(43) Date de publication de la demande: 05.05.2021
(73) Titulaire: ARKEMA FRANCE, 92700 Colombes (FR)
(72) Inventeur: CONOIR, Pierre-Emmanuel, 57501 SAINT AVOLD Cedex (FR)
(74) Mandataire: Arkema Patent
(86) Numéro de dépôt international: PCT/FR2019/051569
(87) Numéro de publication internationale: WO 2020/002830

(56) Documents cités:
- Takashi Ohara ET AL: "Acrylic Acid and Derivatives" In: "Ullmann's Encyclopedia of Industrial Chemistry", 15 octobre 2011 (2011-10-15), Wiley-VCH, Weinheim, XP055569437, ISBN: 978-3-527-30673-2 DOI: 10.1002/14356007.a01_161.pub3, Passage "Lower Alkyl Acrylates" sur pages 8-9 et Figure 4

## Description

### DOMAINE TECHNIQUE

La présente invention concerne la production des esters (méth)acryliques légers tels que le (méth)acrylate de méthyle ou d'éthyle, par estérification directe de l'acide (méth)acrylique par l'alcool léger correspondant.

L'invention a plus particulièrement pour objet un procédé de récupération / purification d'acrylate d'alkyle en C₁-C₂ comprenant la distillation azéotropique du mélange réactionnel brut à l'aide d'une colonne de distillation comprenant un soutirage latéral d'une fraction riche en sous-produit alcoxy propionate d'alkyle dont le point d'ébullition est proche de celui de l'acide acrylique, et donc problématique dans le procédé de purification.

### ART ANTERIEUR ET PROBLEME TECHNIQUE

Il est connu de produire des esters (méth)acryliques en mettant en oeuvre une réaction d'estérification entre un alcool et un acide (méth)acrylique. Cette réaction est une réaction catalysée équilibrée avec génération d'eau. Elle s'accompagne par ailleurs de réactions secondaires produisant des impuretés.

Il est nécessaire d'éliminer l'eau produite pour déplacer l'équilibre, d'éliminer les impuretés, ainsi que de recycler les réactifs non réagis.

A cette fin, on procède généralement à un ensemble de distillations et/ou d'extractions, décantations, qui est à la fois relativement complexe à mettre en œuvre, notamment du fait de la présence de mélanges azéotropes, et coûteux sur le plan énergétique.

Les problèmes qui se posent lors de la fabrication des esters (méth)acryliques légers, en particulier des esters acryliques d'alkyle en C₁-C₂, vont maintenant être exposés, par commodité, sur la base de l'exemple de l'acrylate de méthyle obtenu par estérification de l'acide acrylique par le méthanol. Cependant, les problèmes et la solution proposée par l'invention peuvent être appliqués à la mise en oeuvre de l'éthanol dans la réaction d'estérification.

Au titre de réactions secondaires au cours de la fabrication de l'acrylate de méthyle, l'acide acrylique n'ayant pas réagi peut former des oligomères, tels que l'acide 3-acryloxypropionique (n=2) ou l'acide 3-acryloxy 3-propioxy propionique, (n=3), qui sont des sous-produits lourds de point d'ébullition plus élevé que celui de l'acide acrylique.

Comme autre réaction secondaire, une addition de Michael peut produire des adduits de Michael, en particulier une addition de Michael entre l'acrylate de méthyle déjà formé et le méthanol conduit à la formation de méthoxy propionate de méthyle.

Le méthoxy propionate de méthyle (MPM) est un sous-produit dit lourd car sa température d'ébullition (142°C, P atmosphérique) est nettement supérieure à celle de l'acrylate de méthyle produit (80°C, P atmosphérique), et il se forme en quantité significative dans le procédé au fur et à mesure de l'avancement de la réaction d'estérification en même temps que les oligomères de l'acide acrylique.

Le méthoxy propionate de méthyle pose problème car il présente une tension de vapeur proche de celle de l'acide acrylique. Sa température d'ébullition est proche de celle de l'acide acrylique (144°C, P atmosphérique), et il peut former un azéotrope avec l'eau. Il va se concentrer majoritairement dans la boucle de recyclage de l'acide acrylique n'ayant pas réagi. Il est alors nécessaire d'effectuer une purge de cette boucle de recyclage, pouvant entraîner une perte significative d'acide acrylique. Par ailleurs, le MPM est le plus léger des sous-produits lourds comparativement aux oligomères d'acide acrylique, il peut interférer dans la purification finale de l'acrylate de méthyle et altérer la qualité du produit fini.

T. Ohara et al., "Acrylic acid and Derivatives", dans Ullmann's Encyclopedia of Industrial Chemistry, 2012, divulgue le procédé habituel pour la production d'acrylates d'alkyls inférieurs comme l'acrylate de méthyle.

Afin de limiter la formation de méthoxy propionate de méthyle, il a été proposé dans le document US 6,025,520, d'effectuer la réaction d'estérification sous pression réduite avec un excès d'acide. Ces conditions permettent d'améliorer le rendement et la sélectivité de la réaction d'estérification et de réduire significativement la formation de sous-produits lourds générés par addition de méthanol tels que le méthoxy propionate de méthyle, problématique pour le train de purification de l'ester recherché.

Le procédé décrit dans le document WO 2015/063388 propose de réduire significativement la formation d'alcoxy propionate d'alkyle lors de la synthèse de (méth)acrylate de méthyle ou d'éthyle dans une technologie de réacteur à lit fixe classique en effectuant la réaction d'estérification sous pression atmosphérique, dans des conditions où l'acide est en excès par rapport à l'alcool et où les vitesses volumiques horaires sont élevées.

Il existe toutefois encore un besoin d'éliminer le méthoxy propionate de méthyle formé au cours de la synthèse de l'acrylate de méthyle, préjudiciable pour le bilan matière du procédé (perte de matières premières lors des purges) et pour le train de purification (complexité pour atteindre une pureté élevée).

De façon surprenante, les inventeurs ont découvert qu'il est possible d'éliminer le méthoxy propionate de méthyle par soutirage latéral lors de la distillation azéotropique du mélange réactionnel réalisée dans une unique colonne de distillation munie d'un soutirage latéral.

De plus, les inventeurs ont trouvé que ce soutirage latéral permet aussi de purger une partie de l'eau générée par la réaction d'estérification, favorisant ainsi le déplacement d'équilibre de la réaction pour améliorer le rendement de la réaction, et évitant la formation d'une boucle de recirculation d'eau.

Un des objectifs de la présente invention est donc de fournir un procédé de récupération/purification d'acrylate de méthyle, et plus généralement d'acrylate de méthyle ou d'éthyle, permettant une élimination efficace du méthoxy propionate de méthyle, dans une configuration simplifiée et avec un bilan matières amélioré.

### RESUME DE L'INVENTION

La présente invention a pour objet un procédé de récupération / purification d'acrylate d'alkyle en C₁-C₂ à partir d'un mélange réactionnel issu de l'estérification de l'acide acrylique avec un alcool choisi parmi le méthanol et l'éthanol, caractérisé en ce qu'il comprend la distillation azéotropique dudit mélange réactionnel à l'aide d'une colonne de distillation comprenant un soutirage latéral d'une fraction riche en sous-produit alcoxy propionate d'alkyle.

Par distillation azéotropique, on entend la séparation d'un azéotrope (ou mélange azéotropique) constitué d'un mélange ternaire acrylate d'alkyle / alcool / eau.

Selon un mode de réalisation, l'alcool est le méthanol, l'acrylate d'alkyle est l'acrylate de méthyle, et l'alcoxy propionate d'alkyle est le méthoxy propionate de méthyle (MPM).

Selon un mode de réalisation, l'alcool est l'éthanol, l'acrylate d'alkyle est l'acrylate d'éthyle, et l'alcoxy propionate d'alkyle est l'éthoxy propionate d'éthyle (EPE).

Selon un mode de réalisation, le soutirage de la fraction riche en alcoxy propionate d'alkyle s'effectue en phase liquide.

Selon un mode de réalisation, le soutirage de la fraction riche en alcoxy propionate d'alkyle s'effectue dans la moitié supérieure de la colonne de distillation azéotropique.

Selon un mode de réalisation, la distillation azéotropique est réalisée sous léger vide sous une pression pouvant aller de 26.7 à 80.0 kPa (de 200 à 600 mm de Hg).

Selon un mode de réalisation, la température en pied de la colonne de distillation azéotropique est inférieure à 110°C, de préférence inférieure à 100°C.

Selon un mode de réalisation, un reflux interne est assuré dans la colonne de distillation azéotropique.

Selon un mode de réalisation, un reflux externe est assuré dans la colonne de distillation azéotropique à l'aide d'un flux comprenant de l'acrylate d'alkyle, de préférence à l'aide d'un flux comportant une teneur massique en acrylate d'alkyle supérieure à 90%.

Selon un mode de réalisation, la fraction riche en alcoxy propionate d'alkyle contient de l'alcoxy propionate d'alkyle à une teneur massique pouvant aller d'environ 20% à environ 50%.

Selon un mode de réalisation, la fraction riche en alcoxy propionate d'alkyle contient de l'eau, à une teneur massique pouvant aller d'environ 20% à environ 60%.

Selon un mode de réalisation, le procédé est choisi parmi les procédés de type continu, semi-continu ou discontinu.

Selon un mode de réalisation, le mélange réactionnel est issu de l'estérification de l'acide acrylique avec un excès stoechiométrique d'alcool.

Selon un mode de réalisation, le mélange réactionnel est issu de l'estérification de l'acide acrylique avec un alcool dans des conditions d'excès stoechiométrique d'acide.

Selon un mode de réalisation, le procédé selon l'invention comprend les étapes suivantes :
a) La distillation azéotropique du mélange réactionnel à l'aide d'une première colonne de distillation permettant de séparer en tête un mélange azéotropique comprenant l'acrylate d'alkyle, l'alcool non réagi et de l'eau, et en pied une fraction comprenant l'acide acrylique non réagi et des sous-produits lourds, une fraction riche en sous-produit alcoxy propionate d'alkyle étant éliminée par soutirage latéral ;
b) La séparation du flux de pied de la première colonne de distillation en un flux comportant essentiellement l'acide acrylique non réagi, ce flux étant recyclé dans le réacteur d'estérification, et un flux comprenant essentiellement des sous-produits lourds qui est soumis à un craquage thermique libérant un flux de produits valorisables qui peut être recyclé ;
c) L'extraction liquide / liquide du flux de tête de la première colonne de distillation par un flux aqueux permettant de séparer une phase organique comprenant essentiellement l'acrylate d'alkyle, et une phase aqueuse, la phase aqueuse étant distillée pour récupérer d'une part une fraction riche en alcool qui peut être recyclée vers le réacteur, et d'autre part une fraction riche en eau qui peut être utilisée en tant que flux aqueux dans l'étape d'extraction liquide / liquide ;
d) La purification de ladite phase organique permettant de récupérer l'acrylate d'alkyle purifié.

Selon un mode de réalisation, la purification d) est réalisée par distillation, à l'aide d'un système de purification comprenant au moins une colonne de distillation, de préférence deux colonnes de distillation en série.

Selon un mode de réalisation, la purification d) est effectuée à l'aide d'un système de purification comprenant au moins une colonne à partition.

La présente invention permet de surmonter les inconvénients de l'état de la technique liés à la formation de sous-produit alcoxy propionate d'alkyle dans un procédé de synthèse d'acrylate de méthyle ou d'acrylate d'éthyle par estérification directe de l'acide acrylique avec l'alcool correspondant.

L'invention permet d'éliminer efficacement l'alcoxy propionate d'alkyle, et de réduire les pertes de produits valorisables engendrées par les purges imposées par l'accumulation d'alcoxy propionate d'alkyle dans le train de purification.

L'invention permet également de minimiser l'accumulation d'eau néfaste pour la productivité et la consommation énergétique du procédé.

Cela est accompli grâce à la mise en oeuvre d'une colonne de distillation munie d'un soutirage latéral dans des conditions permettant de faciliter la séparation de l'alcoxy propionate d'alkyle, lors de la distillation azéotropique du mélange réactionnel.

Ainsi l'invention fournit un procédé simplifié de production d'acrylate de méthyle ou d'éthyle de haute pureté, et optimise le bilan matières du procédé.

### BREVE DESCRIPTION DES FIGURES

- La Figure 1 représente de manière schématique une installation pour la mise en oeuvre d'un procédé de production d'acrylate de méthyle selon l'art antérieur.
- La Figure 2 représente de manière schématique une installation pour produire de l'acrylate de méthyle incluant le procédé de récupération/purification selon l'invention.

### EXPOSE DETAILLE DE L'INVENTION

L'invention est maintenant décrite plus en détail et de façon non limitative dans la description qui suit, en comparaison avec un procédé de l'art antérieur.

Pour simplification, la description est basée sur l'exemple de l'acrylate de méthyle obtenu par estérification de l'acide acrylique par le méthanol. Cependant, la solution proposée par l'invention s'applique de la même façon à la mise en oeuvre de l'éthanol dans la réaction d'estérification.

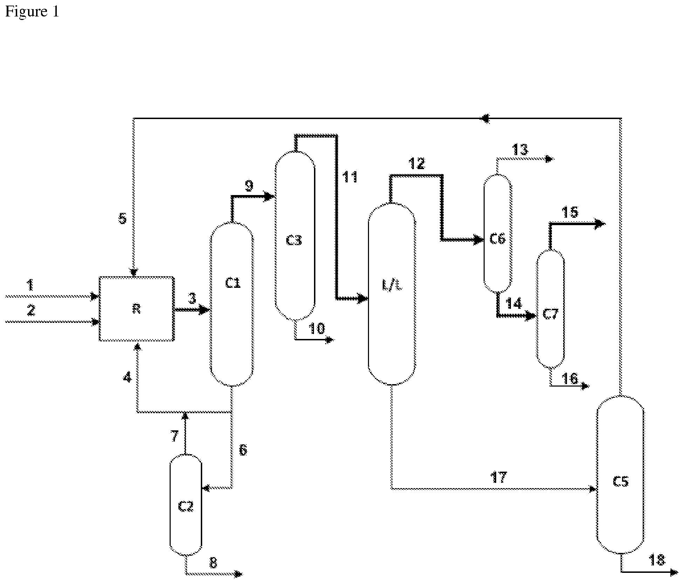

Une installation de production d'acrylate de méthyle de l'art antérieur est représentée sur la **Figure 1****.**

La section réactionnelle comporte un réacteur d'estérification R. Le réacteur R est alimenté par une conduite d'amenée d'acide acrylique 1, une conduite d'amenée de méthanol 2. Le réacteur contient préférentiellement un catalyseur hétérogène de type résine échangeuse de cations acides. Dans le cas d'une catalyse homogène, le réacteur est en outre alimenté par une conduite d'amenée de catalyseur (non représenté). La réaction d'estérification peut être réalisée en excès de méthanol ou en excès d'acide acrylique.

En sortie du réacteur R, le mélange réactionnel 3 est envoyé vers une unité de distillation azéotropique représentée sur la Figure 1 par deux colonnes de distillation en série C1 et C3, la première colonne C1 étant reliée à la seconde colonne C3 par la phase gazeuse 9, et la seconde colonne C3 séparant en pied un flux 10 d'eau constitué d'une partie de l'eau formée par la réaction d'estérification et d'eau injectée sous forme de vapeur dans la colonne C3.

En alternative, l'unité de distillation azéotropique comporte une seule colonne de distillation C1 fonctionnant sous vide dans les mêmes conditions.

La colonne de distillation C1 sépare, en pied, un flux 6 comportant essentiellement l'acide acrylique non réagi, des traces de produits légers (température d'ébullition inférieure à celle de l'acide acrylique), et les produits lourds ayant une température d'ébullition plus élevée que l'acide acrylique (oligomères de l'acide acrylique et adduits de Michael).

Le flux 6 est envoyé vers une colonne de distillation et/ou un évaporateur à film C2 qui sépare un flux 7 comportant l'acide acrylique résiduel et les produits plus légers, et un flux 8 constitué essentiellement des produits lourds. Le flux 7 est avantageusement recyclé dans le réacteur R.

Le flux 8 peut être soumis à un craquage thermique (non représenté sur la Figure 1) permettant de recycler les produits nobles (composés de départ ou produit fini) potentiellement récupérables de la fraction de produits lourds. Le craquage thermique est généralement effectué à une température pouvant aller par exemple de 120°C à 220°C, éventuellement en présnce d'un catalyseur acide tel que l'acide sulfurique ou un acide sulfonique.

L'unité de distillation azéotropique sépare en tête un flux 11 constitué d'un mélange azéotropique comportant l'acrylate de méthyle formé, du méthanol non réagi et l'eau générée par la réaction, ainsi que des impuretés légères et des impuretés lourdes.

Le méthoxy propionate de méthyle MPM généré comme sous-produit lors de la réaction d'estérification, présente la particularité de se retrouver en partie en pied de l'unité azéotropique, et en partie dans le mélange 11 extrait en tête de l'unité azéotropique.

L'élimination complète du MPM présent dans le flux 6 à l'aide de la colonne et/ou l'évaporateur à film C2 est impossible du fait que les tensions de vapeur avec l'acide acrylique sont très proches. Le MPM reste présent en partie dans le flux 7 d'acide acrylique qui est recyclé dans le réacteur, en partie dans le flux 8 qui est soumis à un craquage thermique dont l'effet sur la dissociation du MPM est généralement limité.

Le flux 11 de tête de l'unité de distillation azéotropique est envoyé vers une section d'extraction L/L liquide / liquide (décanteur ou contacteur) qui génère, d'une part, une phase aqueuse 17 contenant essentiellement du méthanol et d'autre part, une phase organique 12.

La section d'extraction liquide / liquide est généralement constituée d'une colonne d'extraction liquide / liquide de type agitée ou colonne garnie, d'une batterie de mélangeur - décanteur, d'un ou plusieurs décanteurs en série.

Dans l'installation décrite sur la Figure 1, la phase aqueuse 17 est soumise à une distillation sur une colonne de distillation C5 pour séparer le méthanol qui est recyclé dans le réacteur (flux 5), le flux aqueux 18 appauvri en méthanol pouvant être recyclé pour la phase d'extraction liquide/liquide.

La phase organique 12 peut être soumise à une ou plusieurs étapes complémentaires de distillation en série. Par exemple une colonne d'étêtage C6 extrait en tête les composés légers résiduels 13, et une colonne d'équeutage C7 sépare en pied un flux 16 comprenant essentiellement les composés lourds résiduels avec de l'acrylate de méthyle.

Un flux 15 d'acrylate de méthyle purifié est extrait en tête de la dernière colonne du train de purification.

Le MPM présent dans le flux 11 extrait en tête de l'unité azéotropique se retrouve finalement mélangé avec de l'acrylate de méthyle en pied de la colonne d'équeutage C7 (flux 16).

Afin d'éviter des pertes en acrylate de méthyle, le flux 16 peut être repris par campagnes sur la colonne et/ou l'évaporateur à film C2 pour séparer et recycler l'acrylate de méthyle et purger le MPM.

Le procédé de production d'acrylate de méthyle selon le schéma représenté sur la Figure 1 est compliqué à mettre en oeuvre et présente l'inconvénient d'accumuler du méthoxy propionate de méthyle à différents endroits du procédé. Il est nécessaire d'effectuer des purges en fin de campagne de production occasionnant des pertes de matières valorisables. Ce type de procédé engendre des pertes importantes d'acrylate de méthyle s'il fonctionne en mode continu.

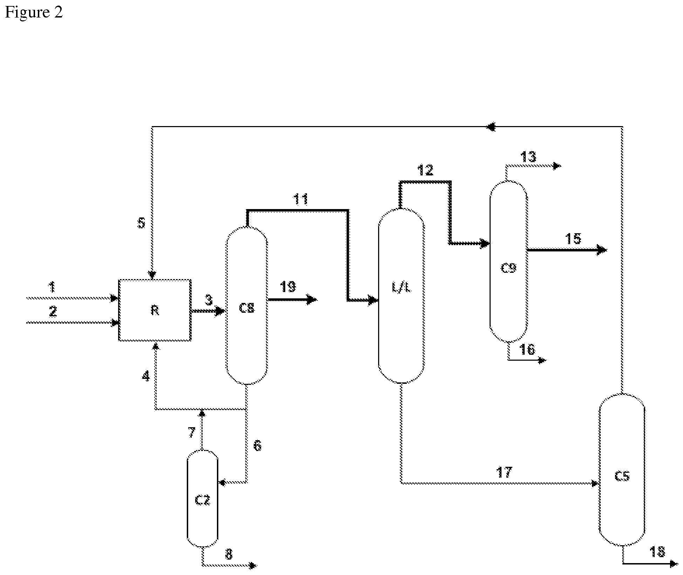

Selon l'invention, l'unité de distillation azéotropique est constituée d'une unique colonne de distillation C8 munie d'un soutirage latéral pour éliminer l'impureté MPM, comme représenté sur la **Figure 2****.**

Comme colonne de distillation C8, on peut utiliser une colonne comprenant des internes du type garnissage vrac ou ordonné du type plateaux dual flow, perforés à déversoir ou à clapets.

Le soutirage latéral 19 est avantageusement placé dans la moitié supérieure de la colonne de distillation.

Le soutirage latéral peut être effectué en phase liquide ou en phase gazeuse ; il est de préférence effectué en phase liquide afin d'optimiser la teneur en MPM extraite tout en limitant le soutirage d'acrylate de méthyle.

La configuration de la colonne de distillation C8 permet de séparer, en tête un flux 11 constitué d'un mélange azéotropique comportant l'acrylate de méthyle formé, du méthanol non réagi et l'eau générée par la réaction, ainsi que des impuretés légères et des impuretés lourdes, en pied un flux 6 comportant essentiellement l'acide acrylique non réagi, des traces de produits légers et des produits lourds, et un flux 19 soutiré latéralement.

Le flux 19 comprend une fraction importante du MPM formé comme sous-produit lors de l'estérification. Le flux 19 comprend en outre une partie de l'eau générée par la réaction d'estérification. Le soutirage latéral permet ainsi de purger une partie de l'eau formée lors de l'estérification. Le soutirage latéral évite l'accumulation de MPM, d'une part dans la section de traitement du flux 6 séparé en pied de la colonne de distillation azéotropique, et d'autre part dans le train de purification du flux 11 comprenant l'acrylate de méthyle séparé en tête de la colonne de distillation azéotropique.

Le flux 19 est un flux riche en MPM, c'est-à-dire qu'il contient environ de 20% à 50% massique de MPM, et peut contenir environ de 20% à 60% massique d'eau. Le flux 19 peut contenir en outre de l'acrylate de méthyle, du méthanol et de l'acide acrylique en faible teneur.

Le soutirage du flux 19 optimise l'élimination du MPM tout en minimisant l'élimination de produits valorisables au sein du procédé dans son ensemble.

Le flux 19 peut avantageusement être utilisé comme diluant de flux de produits lourds, ou pour véhiculer des flux visqueux, ces flux pouvant être soumis à une opération de craquage thermique ou catalytique pour récupérer les composés ainsi valorisables.

En alternative, le flux 19 peut être soumis à une purification afin de récupérer le méthoxy propionate de méthyle purifié d'une part, et les composés valorisables tels que l'acrylate de méthyle, le méthanol et l'acide acrylique d'autre part.

La distillation azéotropique réalisée à l'aide de la colonne de distillation C8 peut être réalisée sous une pression allant de 26.7 à 80.0 kPa (de 200 à 600 mm de Hg), et à une température allant de 50°C à 110°C, de préférence de 60°C à 100°C.

De préférence, la température en pied de la colonne C8 est inférieure à 110°C, en particulier inférieure à 100°C.

La colonne de distillation peut comporter un reflux direct ou un reflux externe (non représentés sur la Figure 2) assuré par un flux comprenant de l'acrylate de méthyle, en particulier par une partie de la fraction organique 12 extraite en tête de l'extracteur Liquide/Liquide situé en aval de la distillation azéotropique. Ledit flux est un flux concentré en acrylate de méthyle, pouvant contenir plus de 90% massique d'acrylate de méthyle.

Le traitement du flux 6 peut s'effectuer sur une colonne de distillation et/ou un évaporateur à film C2 qui sépare un flux 7 comportant l'acide acrylique résiduel et les produits plus légers, et un flux 8 constitué essentiellement des produits lourds qui est généralement soumis à un craquage thermique.

Selon l'invention, le flux 7 d'acide acrylique non réagi qui est séparé sur la colonne et/ou l'évaporateur à film C2 ne contient que de l'ordre de 10% massique de MPM. Le flux 7 est avantageusement recyclé dans le réacteur sans risque d'accumulation de MPM. En outre, le recyclage d'un peu de MPM à la réaction contribue à limiter sa formation dans le réacteur.

Le flux 11 comprenant le mélange azéotropique AM /méthanol/eau est envoyé vers une section d'extraction L/L liquide / liquide (décanteur ou contacteur) qui génère, d'une part, une phase aqueuse 17 contenant essentiellement du méthanol et, d'autre part, une phase organique 12.

La section d'extraction liquide / liquide est généralement constituée d'une colonne d'extraction liquide / liquide de type agitée ou colonne garnie, d'une batterie de mélangeur - décanteur, d'un ou plusieurs décanteurs en série.

La phase aqueuse 17 peut être soumise à une distillation sur une colonne de distillation C5 pour recycler le méthanol dans le réacteur (flux 5), le flux aqueux 18 appauvri en méthanol pouvant alimenter l'extracteur UL (non représenté sur la Figure 2).

La phase organique 12 est soumise à un train de purification afin de récupérer l'acrylate de méthyle avec la pureté nécessaire à son utilisation ultérieure. Généralement, une pureté supérieure à 99,5%, voire supérieure à 99,8% est recherchée.

Pour ce faire, on peut soumettre la phase organique 12 à une ou plusieurs étapes complémentaires de distillation en série, selon les méthodes connues de l'état de l'art représentées sur la Figure 1. Par exemple une colonne d'étêtage C6 extrait en tête les composés légers résiduels 13, et une colonne d'équeutage C7 sépare en pied un flux 16 comprenant essentiellement les composés lourds résiduels avec de l'acrylate de méthyle.

Selon un mode de réalisation de l'invention, la purification de la phase organique 12 est effectuée à l'aide d'un système de purification comprenant au moins une colonne à partition. Sur la Figure 2 est représentée une unique colonne C9 qui sépare, en tête un flux 13 comprenant l'essentiel des composés légers, en pied un flux 16 comprenant l'essentiel des composés lourds, et un flux 15 d'acrylate de méthyle purifié est soutiré latéralement.

Un exemple de système de purification peut comprendre une colonne à partition équipée d'une cloison partielle interne créant des zones de séparation dans la colonne, et associée en pied à un bouilleur unique et en tête à un condenseur unique, et un décanteur placé en sortie du condenseur de tête. La colonne à partition peut comprendre une section commune de rectification au-dessus de la cloison, une section de préfractionnement comportant l'alimentation de la colonne, une section de soutirage séparée de la section de préfractionnement par la cloison comprenant le soutirage de l'ester purifié, et une section commune de rebouillage au-dessous de la cloison.

Un mode de fonctionnement d'un tel système de purification est notamment décrit dans le document WO 2017/125657.

Selon l'invention, le flux 16 comprenant essentiellement les composés lourds résiduels avec de l'acrylate de méthyle, ne contient pratiquement pas de MPM. Le flux 16 peut être avantageusement utilisé pour la préparation de la solution stabilisante injectée en tout point du procédé pour inhiber les réactions de polymérisation sans risque de pollution par le MPM ; Le flux 16 peut être soumis en partie par campagne au traitement sur la colonne et/ou l'évaporateur à film C2 afin de récupérer l'acrylate de méthyle. Le flux 16 peut aussi être recyclé en partie sur la colonne C8.

Le procédé de récupération / purification d'acrylate de méthyle selon l'invention s'applique à un mélange réactionnel 3 issu de l'estérification de l'acide acrylique 1 avec du méthanol 2 dans le réacteur R.

La réaction d'estérification peut être réalisée en excès de méthanol, dans ce cas le rapport molaire acide/alcool est compris entre 0,6 et 1, ou en excès d'acide acrylique, dans ce cas le rapport molaire acide/alcool est compris entre 1,05 et 3, étant entendu que le rapport molaire acide/alcool fait référence aux teneurs en acide et en alcool de l'ensemble des flux alimentant le réacteur d'estérification (flux de produits purs et flux recyclés).

La réaction d'estérification peut être effectuée sous une pression allant de la pression atmosphérique à quelques bars, ou sous pression réduite.

La réaction d'estérification est réalisée en présence d'un catalyseur acide, par exemple une résine échangeuse de cations acides dans le cas d'une catalyse hétérogène ; ou à titre de catalyseur dans le cas d'une catalyse homogène, on peut utiliser par exemple de l'acide sulfurique, ou un acide organique sulfonique, tel que l'acide méthane sulfonique, l'acide para-toluène sulfonique, l'acide benzène sulfonique, l'acide dodécyl sulfonique, ou leurs mélanges. De préférence, la réaction d'estérification est réalisée en catalyse hétérogène, sous pression atmosphérique.

La réaction est généralement effectuée en présence d'un ou plusieurs inhibiteurs de polymérisation qui sont introduits dans le réacteur, à raison de 500 à 5000 ppm par rapport au mélange brut réactionnel. Comme inhibiteurs de polymérisation utilisables, on peut citer par exemple la phénothiazine, l'hydroquinone, l'éther monométhylique d'hydroquinone, le diterbutyl para-crésol (BHT), la paraphénylène diamine, le TEMPO (2,2,6,6-tétraméthyl-1-piperidinyloxy), le di-tertiobutylcatéchol, ou les dérivés du TEMPO, tel que le OH-TEMPO, seuls ou leurs mélanges en toutes proportions. Un ajout supplémentaire d'inhibiteur de polymérisation est généralement effectué au niveau du traitement ultérieur de purification.

L'invention permet de limiter les pertes de matières valorisables telles que l'acide acrylique, l'alcool ou l'acrylate d'alkyle dans un procédé de production d'acrylate d'alkyle léger par estérification directe.

Les exemples suivants illustrent la présente invention et n'ont pas pour but de limiter la portée de l'invention telle que définie par les revendications annexées.

### PARTIE EXPERIMENTALE

Dans les exemples, les pourcentages sont indiqués en poids sauf indication contraire et les abréviations suivantes ont été utilisées :
AA : acide acrylique
AM : Acrylate de méthyle
MPM : méthoxy propionate de méthyle
MeOH : méthanol

### Exemple 1. Purification d'acrylate de méthyle par simulation d'un procédé selon l'invention

Des simulations à l'aide d'un modèle thermodynamique ont été effectuées pour un mélange réactionnel obtenu par réaction d'acide acrylique avec du méthanol en excès, sur une résine cationique acide.

Le débit d'entrée du mélange réactionnel dans le train de purification est ajusté pour assurer une production d'acrylate de méthyle AM de 120t/j environ.

Le débit de méthoxy propionate de méthyle MPM à purger est de l'ordre de 80 kg/h correspondant à la quantité de MPM formée à la réaction à l'allure de 120 t/j.

Deux configurations ont été comparées, elles diffèrent par la mise en oeuvre de la distillation azéotropique :
- Une distillation azéotropique dans une seule colonne sous léger vide (60 kPa, 450 mm de Hg), la colonne comportant 16 étages théoriques et un soutirage latéral situé à l'étage 3 (selon l'invention).
- Une distillation azéotropique dans la même colonne de distillation sous léger vide, la colonne comportant également 16 étages théoriques mais ne comportant pas de soutirage latéral (référence).

Dans ces 2 configurations, le mélange azéotropique de tête de colonne azéotropique est soumis au même traitement d'extraction Liquide/Liquide.

D'après les simulations, une fraction du MPM est purgée via le pied du craqueur thermique situé en pied de la colonne C2, et une fraction du MPM est purgée en pied de la colonne C5 de distillation de la phase aqueuse générée par l'extraction L/L.

Le tableau 1 ci-dessous compare la répartition des différents débits de purge de MPM, exprimés en kg/h, dans les deux configurations.

**Tableau 1**

| | Référence | Invention |
|---|---|---|
| MPM purgé en pied du craqueur | 14,7 | 14,6 |
| MPM purgé en pied de colonne C5 | 13,9 | 2,9 |
| MPM purgé dans le flux 16 en pied de la colonne de purification AM | 53,6 | 0 |
| MPM purgé par soutirage latéral | 0 | 66,9 |
| Total | 82,2 | 84,4 |

Le procédé de référence crée une boucle de MPM importante en aval de la colonne de séparation azéotropique, d'où une proportion de MPM plus importante dans l'eau purgée en pied de la colonne C5.

Le tableau 2 indique le débit et la composition du principal point de purge de MPM dans chacun des deux procédés, respectivement le flux 16 en pied de la colonne C7 dans le procédé de référence et le soutirage latéral de la colonne C8 dans le procédé selon l'invention.

**Tableau 2**

| | Référence | Invention |
|---|---|---|
| Débit global, kg/h | 250 | 200 |
| MPM, % | 21,4 | 33,4 |
| AM, % | 77,2 | 7,9 |
| AA, % | 0,2 | 3,2 |
| MeOH, % | 0 | 15,2 |
| Eau, % | 0 | 39,1 |

Dans le procédé de référence, la simulation indique des pertes importantes d'AM qui est concentré à 77% dans le pied de la colonne d'équeutage C7. Au contraire, le soutirage latéral mis en place sur la colonne de distillation azéotropique contient 33% de MPM, 39% d'eau et des concentrations limitées de produits valorisables.

Le soutirage latéral d'une fraction riche en MPM permet de réduire de plus de 80% la perte en AM par rapport à un procédé de référence, ce qui représente une réduction pour la consommation des matières premières de l'ordre de 20 kg d'acide acrylique et 4 kg de méthanol par tonne d'AM produite, soit un gain économique de l'ordre de 14 euros par tonne d'acrylate de méthyle produite, sans nécessiter de modification onéreuse de l'installation.

### Exemple 2. Purification au moyen d'un pilote mettant en œuvre le procédé selon l'invention

Le pilote utilisé met en oeuvre une colonne de distillation remplie d'éléments de garnissage structuré équivalents à 25 étages théoriques.

Elle est alimentée en partie inférieure avec 10 kg/h de mélange réactionnel synthétique (AA, AM, méthanol, eau, MPM) à environ 50°C. Le reflux est assuré par un débit externe variable d'AM pur et le soutirage latéral est réalisé en partie supérieure. Des stabilisants sont injectés en tête avec le reflux et dans le condenseur (4OH TEMPO), ainsi que dans l'alimentation (PTZ).

La pression en tête de colonne est fixée à 60 kPa (450 mm de Hg).

Les paramètres d'action principaux sont la quantité d'énergie apportée au rebouilleur de la colonne et le débit de reflux d'AM pur. Ils sont modifiés dans le but de maximiser la concentration de MPM dans le soutirage latéral tout en limitant la concentration d'AA en tête de colonne.

L'analyse des compositions des flux analysés (alimentation, tête de colonne, soutirage latéral et pied de colonne) montre que :
- la quasi-totalité de l'AA présent dans l'alimentation passe en pied de colonne et la tête de colonne contient entre 100 et 1 000 pm d'AA ;
- le MPM est un peu plus léger et jusqu'à 20% du MPM présent dans l'alimentation passe en tête et dans le soutirage latéral, selon les conditions de fonctionnement de la colonne ;
- le flux de pied de colonne contient 10 à 20% d'eau. ;
- le pied de colonne contient très peu d'AM (500 à 2 000 ppm) et de méthanol (2 000 à 6 000 ppm). Ces deux espèces passent très majoritairement en tête et dans le soutirage latéral.

Les résultats ainsi obtenus ont été regroupés dans le Tableau 3. Ces résultats montrent que le soutirage latéral est enrichi en MPM et pauvre en composés valorisables (AM, méthanol, AA). Par ailleurs, ils valident les résultats de l'exemple 1, obtenus par simulation.

**Tableau 3**

| | Alimentation | Reflux | Tête | Pied | Soutirage latéral |
|---|---|---|---|---|---|
| Débit (kg/h) | 10,0 | 5,7 | 10,7 | 5,0 | 0,03 |
| Méthanol | 12,2% | | 11,5% | 0,5% | 9,8% |
| AM | 32,5% | 100% | 83,0% | 0,2% | 8,6% |
| MPM | 8,5% | | 0,7% | 14,3% | 38,2% |
| AA | 33,3% | | 0,1% | 67,8% | 0,1% |
| H2O | 13,4% | | 4,7% | 17,2% | 43,3% |

## Revendications

1. Procédé de récupération / purification d'acrylate d'alkyle en C₁-C₂ à partir d'un mélange réactionnel issu de l'estérification de l'acide acrylique avec un alcool choisi parmi le méthanol et l'éthanol, **caractérisé en ce qu'**il comprend la distillation azéotropique dudit mélange réactionnel à l'aide d'une colonne de distillation comprenant un soutirage latéral d'une fraction riche en sous-produit alcoxy propionate d'alkyle.

2. Procédé selon la revendication 1 **caractérisé en ce que** le soutirage latéral de la fraction riche en alcoxy propionate d'alkyle s'effectue en phase liquide.

3. Procédé selon la revendication 1 ou 2 **caractérisé en ce que** le soutirage de la fraction riche en alcoxy propionate d'alkyle s'effectue dans la moitié supérieure de la colonne de distillation azéotropique.

4. Procédé selon l'une quelconque des revendications précédentes **caractérisé en ce que** la distillation azéotropique est réalisée sous léger vide sous une pression pouvant aller de 26.7 à 80.0 kPa (de 200 à 600 mm de Hg).

5. Procédé selon l'une quelconque des revendications précédentes **caractérisé en ce que** la température en pied de la colonne de distillation azéotropique est inférieure à 110°C, de préférence inférieure à 100°C.

6. Procédé selon l'une quelconque des revendications précédentes **caractérisé en ce qu'**un reflux externe est assuré dans la colonne de distillation azéotropique à l'aide d'un flux comprenant de l'acrylate d'alkyle.

7. Procédé selon l'une quelconque des revendications précédentes **caractérisé en ce que** la fraction riche en alcoxy propionate d'alkyle contient de l'alcoxy propionate d'alkyle à une teneur massique pouvant aller de 20% à 50%.

8. Procédé selon l'une quelconque des revendications précédentes **caractérisé en ce que** la fraction riche en alcoxy propionate d'alkyle contient de l'eau, à une teneur massique pouvant aller de 20% à 60%.

9. Procédé selon l'une quelconque des revendications précédentes **caractérisé en ce qu'**il est choisi parmi les procédés de type continu, semi-continu ou discontinu.

10. Procédé selon l'une quelconque des revendications précédentes **caractérisé en ce qu'**il comprend les étapes suivantes :
a) La distillation azéotropique du mélange réactionnel à l'aide d'une première colonne de distillation permettant de séparer en tête un mélange azéotropique comprenant l'acrylate d'alkyle, l'alcool non réagi et de l'eau, et en pied une fraction comprenant l'acide acrylique non réagi et des sous-produits lourds, une fraction riche en sous-produit alcoxy propionate d'alkyle étant éliminée par soutirage latéral ;
b) La séparation du flux de pied de la première colonne de distillation en un flux comportant essentiellement l'acide acrylique non réagi, ce flux étant recyclé dans le réacteur d'estérification, et un flux comprenant essentiellement des sous-produits lourds qui est soumis à un craquage thermique libérant un flux de produits valorisables qui peut être recyclé ;
c) L'extraction liquide / liquide du flux de tête de la première colonne de distillation par un flux aqueux permettant de séparer une phase organique comprenant essentiellement l'acrylate d'alkyle, et une phase aqueuse, la phase aqueuse étant distillée pour récupérer d'une part une fraction riche en alcool qui peut être recyclée vers le réacteur, et d'autre part une fraction riche en eau qui peut être utilisée en tant que flux aqueux dans l'étape d'extraction liquide / liquide ;
d) La purification de ladite phase organique permettant de récupérer l'acrylate d'alkyle purifié.

11. Procédé selon la revendication 10 **caractérisé en ce que** la purification d) est réalisée par distillation, à l'aide d'un système de purification comprenant au moins une colonne de distillation, de préférence deux colonnes de distillation en série.

12. Procédé selon la revendication 10 **caractérisé en ce que** la purification d) est réalisée à l'aide d'un système de purification comprenant au moins une colonne à partition.

13. Procédé selon l'une quelconque des revendications précédentes **caractérisé en ce que** l'acrylate d'alkyle est l'acrylate de méthyle.

## Patentansprüche

1. Verfahren zur Gewinnung / Aufreinigung von C₁-C₂-Alkylacrylat ausgehend von einer Reaktionsmischung, die aus der Veresterung von Acrylsäure mit einem Alkohol hervorgeht, welcher aus Methanol und Ethanol ausgewählt ist, **dadurch gekennzeichnet, dass** es die azeotrope Destillation der Reaktionsmischung mit Hilfe einer Destillationskolonne umfasst, welche eine seitliche Entnahme einer Fraktion umfasst, die reich an dem Nebenprodukt Alkylalkoxypropionat ist.

2. Verfahren nach Anspruch 1, **dadurch gekennzeichnet, dass** die seitliche Entnahme der Fraktion, die reich an Alkylalkoxypropionat ist, in der Flüssigphase erfolgt.

3. Verfahren nach Anspruch 1 oder 2, **dadurch gekennzeichnet, dass** die Entnahme der Fraktion, die reich an Alkylalkoxypropionat ist, in der oberen Hälfte der azeotropen Destillationskolonne erfolgt.

4. Verfahren nach einem beliebigen der vorhergehenden Ansprüche, **dadurch gekennzeichnet, dass** die azeotrope Destillation unter einem leichten Vakuum durchgeführt wird, wobei der Druck im Bereich von 26,7 bis 80,0 kPa (von 200 bis 600 mm Hg) liegen kann.

5. Verfahren nach einem beliebigen der vorhergehenden Ansprüche, **dadurch gekennzeichnet, dass** die sumpfseitige Temperatur der azeotropen Destillationskolonne niedriger als 110 °C, vorzugsweise niedriger als 100 °C ist.

6. Verfahren nach einem beliebigen der vorhergehenden Ansprüche, **dadurch gekennzeichnet, dass** in der azeotropen Destillationskolonne ein externer Rückfluss mit Hilfe eines Stoffstroms sichergestellt wird, welcher Alkylacrylat enthält.

7. Verfahren nach einem beliebigen der vorhergehenden Ansprüche, **dadurch gekennzeichnet, dass** die Fraktion, welche reich an Alkylalkoxypropionat ist, Alkylalkoxypropionat zu einem Massenanteil enthält, der im Bereich von 20 % bis 50 % liegen kann.

8. Verfahren nach einem beliebigen der vorhergehenden Ansprüche, **dadurch gekennzeichnet, dass** die Fraktion, welche reich an Alkylalkoxypropionat ist, Wasser enthält, zu einem Massenanteil, der im Bereich von 20 % bis 60 % liegen kann.

9. Verfahren nach einem beliebigen der vorhergehenden Ansprüche, **dadurch gekennzeichnet, dass** es aus den Verfahren kontinuierlicher, halbkontinuierlicher oder diskontinuierlicher Art ausgewählt ist.

10. Verfahren nach einem beliebigen der vorhergehenden Ansprüche, **dadurch gekennzeichnet, dass** es die folgenden Schritte umfasst:
a) Azeotrope Destillation der Reaktionsmischung mit Hilfe einer ersten Destillationskolonne, die es ermöglicht, kopfseitig ein azeotropes Gemisch, welches Alkylacrylat, nicht umgesetzten Alkohol und Wasser umfasst, und sumpfseitig eine Fraktion abzutrennen, die nicht umgesetzte Acrylsäure und schwerflüchtige Nebenprodukte umfasst, wobei eine Fraktion, die reich an dem Nebenprodukt Alkylalkoxypropionat ist, durch seitliche Entnahme entfernt wird;
b) Auftrennung des sumpfseitigen Stoffstroms der ersten Destillationskolonne in einen Stoffstrom, der im Wesentlichen nicht umgesetzte Acrylsäure umfasst, wobei dieser Stoffstrom in den Veresterungsreaktor zurückgeführt wird, und einen Stoffstrom, der im Wesentlichen schwerflüchtige Nebenprodukte umfasst, wobei er einem thermischen Crackvorgang unterzogen wird, welcher einen Stoffstrom an verwertbaren Produkten freisetzt, der zurückgeführt werden kann;
c) Flüssig/flüssig-Extraktion des kopfseitigen Stoffstroms der ersten Destillationskolonne mit einem wässrigen Stoffstrom, wodurch es ermöglicht wird, eine organische Phase, die im Wesentlichen Alkylacrylat umfasst, und eine wässrige Phase abzutrennen, wobei die wässrige Phase destilliert wird, um einerseits eine alkoholreiche Phase, welche zum Reaktor zurückgeführt werden kann, und andererseits eine wasserreiche Fraktion zu gewinnen, welche als wässriger Stoffstrom im Schritt der Flüssig/flüssig-Extraktion verwendet werden kann;
d) Aufreinigung der organischen Phase, wodurch es ermöglicht wird, aufgereinigtes Alkylacrylat zu gewinnen.

11. Verfahren nach Anspruch 10, **dadurch gekennzeichnet, dass** die Aufreinigung d) durch Destillation erfolgt, mit Hilfe eines Aufreinigungssystems, das mindestens eine Destillationskolonne, vorzugsweise zwei hintereinander angeordnete Destillationskolonnen umfasst.

12. Verfahren nach Anspruch 10, **dadurch gekennzeichnet, dass** die Aufreinigung d) mit Hilfe eines Aufreinigungssystems erfolgt, welches mindestens eine Kolonne mit Scheidewand umfasst.

13. Verfahren nach einem beliebigen der vorhergehenden Ansprüche, **dadurch gekennzeichnet, dass** es sich bei dem Alkylacrylat um Methylacrylat handelt.

## Claims

1. A process for the recovery/purification of C₁-C₂ alkyl acrylate from a reaction mixture resulting from the esterification of acrylic acid with an alcohol chosen from methanol and ethanol, **characterized in that** it comprises the azeotropic distillation of said reaction mixture using a distillation column comprising a sidestream drawoff of a fraction rich in alkyl alkoxypropionate byproduct.

2. The process as claimed in claim 1, **characterized in that** the sidestream drawing off of the fraction rich in alkyl alkoxypropionate is carried out in the liquid phase.

3. The process as claimed in claim 1 or 2, **characterized in that** the drawing off of the fraction rich in alkyl alkoxypropionate is carried out in the upper half of the azeotropic distillation column.

4. The process as claimed in any one of the preceding claims, **characterized in that** the azeotropic distillation is carried out under a slight vacuum under a pressure which can range from 26.7 to 80.0 kPa (200 to 600 mmHg).

5. The process as claimed in any one of the preceding claims, **characterized in that** the temperature at the bottom of the azeotropic distillation column is less than 110°C, preferably less than 100°C.

6. The process as claimed in any one of the preceding claims, **characterized in that** an external reflux is provided in the azeotropic distillation column using a stream comprising alkyl acrylate.

7. The process as claimed in any one of the preceding claims, **characterized in that** the fraction rich in alkyl alkoxypropionate contains alkyl alkoxypropionate at a content by weight which can range from 20% to 50%.

8. The process as claimed in any one of the preceding claims, **characterized in that** the fraction rich in alkyl alkoxypropionate contains water at a content by weight which can range from 20% to 60%.

9. The process as claimed in any one of the preceding claims, **characterized in that** it is chosen from processes of continuous, semi-continuous or batch type.

10. The process as claimed in any one of the preceding claims, **characterized in that** it comprises the following stages:
a) the azeotropic distillation of the reaction mixture using a first distillation column making it possible to separate, at the top, an azeotropic mixture comprising the alkyl acrylate, the unreacted alcohol and water and, at the bottom, a fraction comprising the unreacted acrylic acid and heavy byproducts, a fraction rich in alkyl alkoxypropionate byproduct being removed by sidestream drawoff;
b) the separation of the bottom stream from the first distillation column into a stream comprising essentially the unreacted acrylic acid, this stream being recycled to the esterification reactor, and a stream comprising essentially heavy byproducts, which is subjected to a thermal cracking releasing a stream of economically upgradable products, which stream can be recycled;
c) the liquid/liquid extraction of the top stream from the first distillation column with an aqueous stream making it possible to separate an organic phase, comprising essentially the alkyl acrylate, and an aqueous phase, the aqueous phase being distilled in order to recover, on the one hand, a fraction rich in alcohol which can be recycled to the reactor and, on the other hand, a fraction rich in water which can be used as aqueous stream in the liquid/liquid extraction stage;
d) the purification of said organic phase making it possible to recover the purified alkyl acrylate.

11. The process as claimed in claim 10, **characterized in that** the purification d) is carried out by distillation, using a purification system comprising at least one distillation column, preferably two distillation columns in series.

12. The process as claimed in claim 10, **characterized in that** the purification d) is carried out using a purification system comprising at least one partition column.

13. The process as claimed in any one of the preceding claims, **characterized in that** the alkyl acrylate is methyl acrylate.
